Europäisches Patentamt

European Patent Office

Office européen des brevets

(11) Publication number: **0 352 972**
**A2**

(12) **EUROPEAN PATENT APPLICATION**

(21) Application number: 89307386.6

(51) Int. Cl.4: **A61L 27/00 , A61F 2/06**

(22) Date of filing: 20.07.89

(30) Priority: 29.07.88 US 227973

(43) Date of publication of application:
**31.01.90 Bulletin 90/05**

(84) Designated Contracting States:
**DE FR GB**

(71) Applicant: BAXTER INTERNATIONAL INC. (a
Delaware corporation)
One Baxter Parkway
Deerfield Illinois 60015(US)

(72) Inventor: **Tu, Roger**
**24755 Scott Lane**
**Lake Forest, CA 92630(US)**
Inventor: **Wang, Edwin**
**10 Varesa**
**Irvine, CA 92720(US)**

(74) Representative: MacGregor, Gordon et al
ERIC POTTER & CLARKSON 14 Oxford Street
Nottingham, NG1 5BP(GB)

(54) **Fiber-reinforced expanded fluoroplastic vascular grafts.**

(57) A composition of matter which comprises a biocompatible fluoroplastic resin which is impregnated with biocompatible fibers in a random orientation, a vascular graft comprised of the disclosed composition and processes for manufacturing the composition and the vascular graft.

EP 0 352 972 A2

## FIBER-REINFORCED EXPANDED FLUOROPLASTIC VASCULAR GRAFTS

### Background of the Invention

### Field of the Invention

This invention relates to fiber-reinforced expanded fluoroplastic compositions useful for making vascular grafts.

### Description of the Relevant Art

In recent years, a variety of materials have been used to make prosthetic veins and arteries. Prostheses have been made, for example, using woven or knitted fabric made from Dacron or polytetrafluoroethylene (PTFE). When these prostheses are constructed with relatively large inner diameters, typically at least about 7 mm, they have proved to be relatively successful. Attempts to make similar prostheses with smaller inner diameters, however, have not been as successful, primarily because it has not been possible to inhibit platelet adhesion to the inner surface of the artificial veins and allow sufficient blood flow.

Prostheses also have been made of stretched or expanded PTFE. A number of references describe such tubing, including Soyer et al., "A New Venous Prosthesis", Surgery, Vol. 72, pages 864 (1972). Volder et al., "A-V Shunts created in New Ways", Trans. Amer. Soc. Artif. Int. Organs, Vol. 19, p. 38 (1973), Application of Expanded Polytetrafluoroethylene to Artificial Vessels", Artificial Organs, Vol. 1, p. 44 (1972), Ibid., Vol. 2, p. 262 (1973), and Ibid., Vol. 3, p. 50," Replacement of the Superior and Inferior Venae Canal", The Journal of Thoracic and Cardiovascular Surgery, Vol. 67, p. 774 (1974), and Goldfarb, Belgian Pat. No. 517,415.

PTFE tubing which has been stretched is characterized by a porous microstructure of fibers and nodes connected together by the fibers. Fiber diameter can vary depending upon the stretching conditions and can be made much smaller than the diameter of a fiber in the knitted and woven fabrics discussed above.

An advantage to using conventional vascular grafts made from expanded PTFE is that they do not suffer significant occlusion by fibrin deposition, in contrast to the non-expanded PTFE grafts. Grafts comprising expanded PTFE have not proved wholly satisfactory, however, because if the prosthesis has too high a porosity, it can be torn by the sutures used to join the prosthesis with the patient's blood vessel. Conventional grafts of expanded PTFE also have strength limitations. The products typically are made by expanding or stretching them uniaxially. See, for example, U.S. Patent No. 3,953,566. When the grafts are attached to a patient's blood vessel, the sutures have a pulling direction that is parallel to the PTFE fibrils, resulting in a low suture retention strength.

To compensate for this lack of suture retention strength, a variety of different types of grafts have been tried. For example, an expanded PTFE has been manufactured with a reinforcing film (e.g. a Gore-Tex graft as described in Jack A. Cannon, Chap. 5 "The Expanded Reinforced Polytetrafluoroethylene Prosthetic Vascular Graft", pp. 31-42 in the book Vascular Grafting, edited by Creighton B. Wright et al., (1983)). It has been found however, that the use of such a product results in a slowing of the desired ingrowth of the perigraft tissue. Furthermore, the outside reinforcing film has a tendency to separate from the expanded PTFE tubing. A graft having an extremely thick wall also has been tried, as described in John M. Johnson et al., The American Journal of Surgery, 132:723-727 (1976), but its use resulted in the same slowing of the desired perigraft tissue ingrowth for healing. A graft with a repeating pattern of external ribs, described in U.S. Patent No. 4,550,447, also has been used, but it too caused a slowing of perigraft tissue ingrowth. U.S. Patent 4,208,745 discloses a vascular prosthesis of PTFE tubing having a fibrous structure that is finer at its inner surface than at its outer surface. A drawback to this type of prosthesis, however, is that the process for its manufacture requires complex, specialized equipment.

In view of the shortcomings of the vascular grafts known in the art, a need exists for a vascular graft that has increased suture retention strength, does not retard the healing process of perigraft tissue ingrowth and is uncomplicated to manufacture.

### Summary of the Invention

The present invention provides a porous composition suitable for use as a vascular graft which resists tearing by the sutures attaching it to perigraft material. The composition comprises an expanded biocompatible fluoroplastic resin and biocompatible, high temperature-resistant fibers which are chemically compatible with the PTFE resin, wherein the fibers are distributed throughout the resin in a random orientation. The composition is made by mixing the fibers with the PTFE resin, then preforming, extruding, expanding and optionally sintering the resulting mixture. The composition made by this method retains the desirable characteristics of conventional PTFE vascular grafts, yet has the additional desired features of increased burst strength, suture retention, and tensile strength. In a preferred embodiment, the high temperature-resistant fibers comprise PTFE or expanded PTFE.

### Brief Description of the Drawings

Figure 1 is a scanning electron microphotograph of the lumen (inner) surface of fiber-reinforced polytetrafluoroethylene material made in accordance with the present invention.

Figure 2 is a scanning electron microphotograph of the outer, or outside, surface of the fiber-reinforced PTFE material made in accordance with the present invention.

Figure 3 is a scanning electron microphotograph of a cross-section of the fiber-reinforced PTFE material made in accordance with the present invention.

Figure 4 is a schematic view of a graft made in accordance with this invention operating upon attaching sutures.

### Definitions

As an aid to understanding the present specification the following terms are particularly defined. "Biocompatible" means non-antigenic and capable of being implanted within a body without substantial deleterious effects on surrounding tissue.

"Chemically compatible" means that a specified substance is capable of cohering to another specified substance over a wide range of temperature and pressure conditions.

"Chemically distinct" means that a specified substance has a distinct chemical composition from another specified substance. A chemically distinct substance may be chemically compatible with a substance it is distinct from. In the present invention, for example, aramids, ultrahigh molecular weight polyethylenes, polyesters, silicones, polyurethanes and elastomers are all chemically distinct from PTFE, but are chemically compatible with PTFE.

"Perigraft tissue" is the vascular tissue which forms adjacent to the outside surface of the graft upon implantation in the host body.

"Thin-walled" refers to a vascular graft wall with a thickness of from about 0.2 mm to about 0.8 mm.

### Detailed Description of the Invention

This invention is directed to compositions, useful in in vivo implantation as vascular grafts, which are prepared from a biocompatible fluoroplastic resin binder which has been reinforced with randomly oriented biocompatible fibers. It is preferred that the resin binder be polytetrafluorethylene and further that this PTFE be expanded. Any type of polytetrafluroethylene may be used in this invention, with those having a molecular weight of about 500,000 to about 1,000,000 being preferred.

The fibers, in addition to being from a biocompatible material, are characterized by being chemically compatible with the resin. When the composition is to be sintered, as is preferred, the fibers also are selected on the basis of their ability to survive exposure to high temperatures. More particularly, when the fluoroplastic resin comprises PTFE, the fibers desirably can withstand exposure to a temperature of greater than about 342° C. Suitable fibers include PTFE fibers, expanded PTFE fibers, aramids, such as an aromatic polyamide (e.g. Kevlar 49® manufactured by E.I. du Pont de Nemours and Company), ultrahigh molecular weight polyethylene fibers such as the Spectrum® series manufactured by Allied Corporation, Dacron® series fibers, or elastomer fibers such as silicone or polyurethane, or a combination of the above. Kalrez®, a perfluorinated elastomeric copolymer of tetrafluoroethylene and perfluoro (methyl vinyl ether), manufactured by E. I. dupont de Nemours and Company, is one example of a suitable elastomer.

The reinforcing fibers are blended into the PTFE resin in an amount sufficient to reinforce the resin. Generally, the fibers comprise a weight percentage of from about .0005% to about 10% of the resin-fiber composition, depending upon the desired application of the final composition. The fiber diameter desirably ranges from about one micron to about 50 microns, preferably from 5 to 25 microns. When the fiber diameter is about the same order of magnitude as the diameter of the PTFE nodes, better contact between the fibers and PTFE results. A desirable fiber length is from about 1 mm to about 5 cm, preferably from 4 mm to 2 cm. Generally, if the reinforcing fiber is too short, then little reinforcement in the form of suture retention is gained. On the other hand, if the reinforcing fiber is too long, then it may cut through the surfaces during processing, thereby providing a locus for platelet adhesion. In a commercial embodiment, a typical fiber length is about 1/2 cm in an individual monofilament.

The fibers are added to the resin such that the basic PTFE fibril-nodal matrix is not altered. It is highly desirable that this matrix be retained, for it is known in the art that this fibril-nodal matrix is conducive to perigraft tissue ingrowth. Such ingrowth is essential to the healing process. In a preferred embodiment for making the reinforced PTFE resins, commercially available PTFE powder is lubricated with about 15% to 40% volume of lubricants known to persons skilled in the art. Suitable lubricants include mineral oil, liquid paraffin, naptha, etc. Generally the PTFE powder has a particle size of from 0.1 to about 0.5 microns and a surface area of about 5 to about 15 m²/g. The lubricated powder then is agitated. The agitation may occur by methods known in the art such as tumbling. While it is being agitated, the desired amount of fiber is added. This blend then is preferably loaded into a preformer and preformed under pressure. After preforming, the material may be extruded in the form of tubular, thin wall extrudates. Any extrude known in the art, such as a ram-type extruder, can be used. The internal diameter of these thin wall extrudates can be selected according to the desired final use of the material. Alternatively, the powder with the added fibers may be molded or rolled before extrusion.

The extrudate next undergoes expansion according to methods known in the art. Expansion may range from about 110% to about 800% of the length of the original extrudate. Preferably, the expansion of the extrudate is about 400%. The stretching which is a result of this expansion process produces the porosity of the structure that is so desirable for perigraft tissue ingrowth. Generally, the material is stretched such that it has a porosity of from about 20% to about 90%, preferably from about 40% to about 70%. Preferably, this expanded material then is sintered by means known in the art.

The foregoing steps result in a fiber-reinforced resin composition useful for vascular grafts. The randomly-oriented fibers confer burst resistance to the graft, thus reducing the likelihood of failure by aneurysm. Because the fibers confer additional strength, increasing the weight percentage of fibers in the composition allows the manufacture of grafts with thinner-walls and higher strengths than conventional grafts. This provides an important benefit for small diameter vascular grafts, which heretofore have proved difficult to construct.

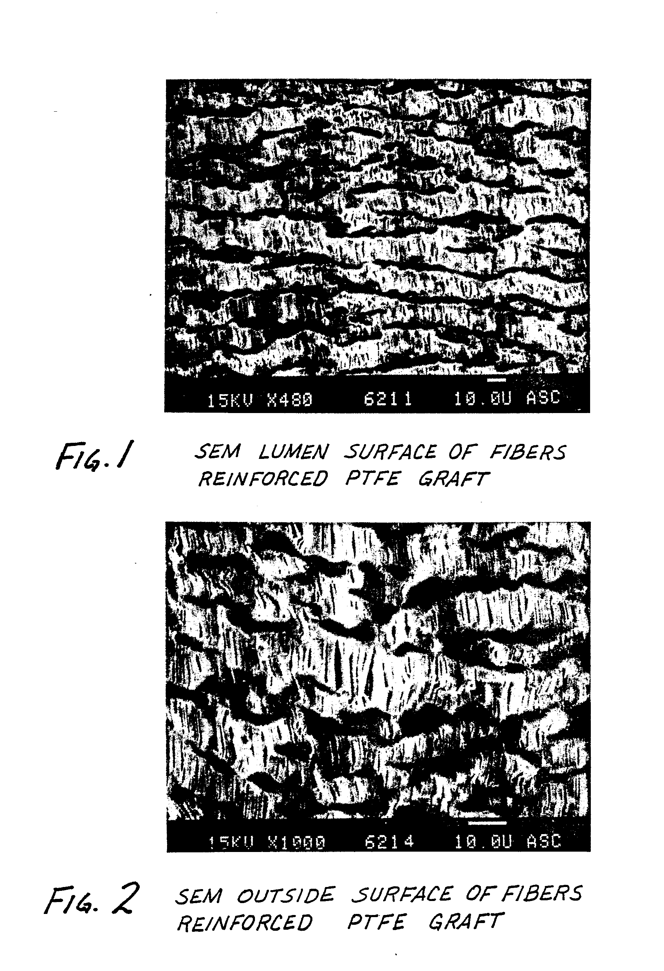

Grafts constructed in accordance with the present invention are shown in the electron microphotographs of Figures 1, 2 and 3.

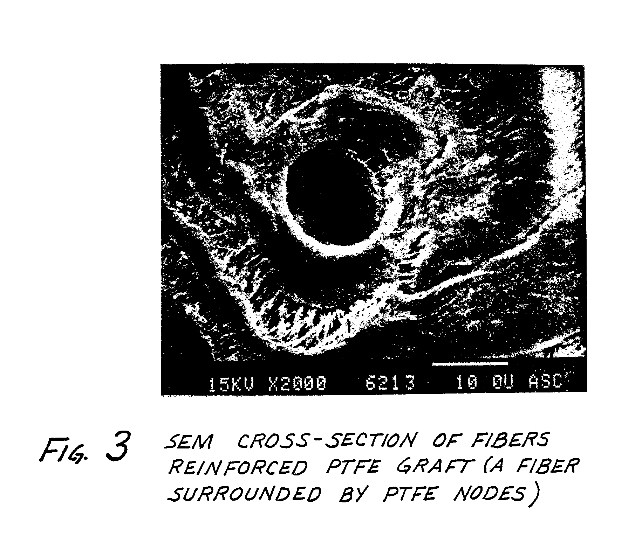

Figures 1 and 2 are scanning electron microphotographs, at 480x and 1000x respectively, of the inside surface (1) and the outside surface (2) of a PTFE graft reinforced with an aromatic polyamide fiber (Kevlar 49, manufactured by E.I. du Pont de Nemours and Company) constructed in accordance with the method of Example 1, below. The photograph shows a typical PTFE fibril (3)-nodal (4) microstructure. No trace of the reinforcing fibers is observed at either surface. Therefore, no adverse tissue reaction, between fibers and adjacent tissue, at either surface, is expected and no obstruction to tissue ingrowth occurs.

Figure 3 is a scanning electron microphotograph at 2000x, of the cross-section of the interior of the wall of a PTFE graft reinforced with the fiber described in the description of Figure 1. This fiber (5) is randomly oriented within the tubing circumferentially. It is surrounded by PTFE nodes (4) and fibrils (3). As can be seen (6) the basic fibril-nodal matrix has been slightly dislocated in the immediate vicinity of the fiber (5). The fiber (5) has a diameter of 12 microns.

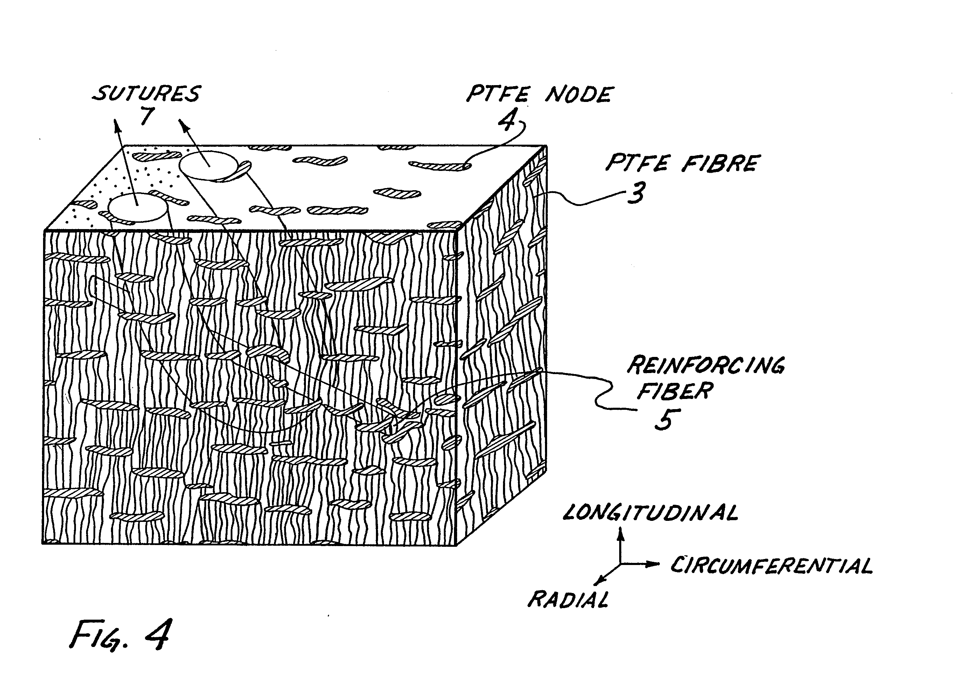

In addition to increased burst strength, the fiber-reinforced vascular grafts of this invention have an increased suture retention strength. Implantation procedures involve suturing of the vascular graft to the native vessels. See Figure 4, which is a schematic diagram of a section of fiber - reinforced PTFE as it interacts with a penetrating suture (7). The fiber (5) is randomly oriented. The suture (7) loops around the fiber (5) during suturing. A suture looping around an embedded fiber will be secured more firmly than one located in a non-reinforced PTFE vascular graft or in a reinforced PTFE vascular graft in which the reinforcing fibers align with the length of the resin tube. Without wishing to be bound by theory, it is believed that this increased strength is due to the nodal concentration of PTFE surrounding the fibers, as can be seen in Figure 3. Manipulation of the amount and type of fibers will affect the suture retention strength. This strength is dependent upon the relative location of a suture to the fiber end, the orientation of the fibers (random orientation increases the likelihood of a suture looping around a fiber), the length of the fibers, the number of fibers holding said suture and the cohesive force between the fiber and the reisn matrix. One reason for preferring the use of PTFE or expanded PTFE fibers with a PTFE resin matrix is the strength of the cohesive force between the matrix and fibers, when identical materials are used.

The following examples are provided for illustrative purposes and are not to be construed as limited.


Example 1. Graft Preparation


Kevlar 49® is an aromatic polyamide fiber manufactured by E. I. du Pont de Nemours and Company. It has the following properties:

Diameter = 12 microns
Specific gravity = 1.45
Tensile strength = 400,000 psi
Tensile modulus = $19 \times 10^6$ psi
Elongation = 2.4%
Decomposition temperature = 500° C

PTFE fiber is available from du Pont as continuous multifilament yarn staple flock or tow. The PTFE fiber has the following properties

Diameter = 20 microns
Specific gravity = 2.1
Tensile strength = 52,500
Tensile modulus = $2.8 \times 10^5$ psi
Elongation = 19%

PTFE powder (Fluon® CD 123 from ICI Americas Inc.) was tumbled in a glass jar with about 20% mineral spirit as lubricant. In half of the lubricated PTFE powder Kevlar 49® fibers (about $\frac{1}{2}$ cm long in an individual monofilament form) at 0.01 wt% based on the PTFE powder were added and sparsely mixed. The lubricated PTFE powder was first loaded into a preformer while the lubricated fiber plus PTFE powder was loaded into the upper portion of the preformer. After preforming under pressure the billet was extruded to form nominal 6 mm internal diameter thin wall extrudates. The extrudates without fibers were used as control. The extrudates then were expanded at 600° F, in an oven, to 400% of their original length. The subsequent sintering conditions were 350° C for 3 minutes.

Figure 3 is a cross-sectional view of this composition shown under a scanning electron microscope at a magnification of 2000X. Note the nodal concentration of PTFE surrounding the fiber. This adherence provides the suture retentive force as is shown schematically in figure 4. Both fiber reinforced expanded PTFE and control expanded PTFE showed similar physical properties of

internal diameter = 5.3 mm
outside diameter = 5.8 mm
density = 0.9 g/cc
porosity = 60%

Due to the small amount of Kevlar® fibers added, both samples appeared and felt similar to each other. The control expanded PTFE had a suture retention strength (by using a 6.0 Prolene suture at 0.2 mm from the edge) of about 11 pounds per inch wall thickness. The fiber reinforced expanded PTFE showed a suture retention strength of 31 pounds per inch wall thickness, about 3 times of the control strength.

Example 2.

Kalrez® is a perfluorinated elastomeric copolymer of tetrafluorethylene and perfluoro (methyl vinyl ether) manufactured by E.I. du Pont de Nemours and Company. Kalrez® fibers can be produced via melt spinning. Such fibers have the following general properties:

Specific gravity = 2.0
Critical surface tension = 19.1 dynes/cm
Tensile strength = 2720 psi
Tensile modulus = 1350 psi
Elongation = 160%
Hardness = 89 durometer A
Decomposition temperature = 400° C

By using Kalrez® in place of Kevlar 49® in the method of Example 1, substantially the same results are obtained.

## Claims

1. A composition of matter comprising
a) an expanded, biocompatible fluoroplastic resin and
b) biocompatible, high temperature resistant fibers which are chemically distinct from, but chemically compatible with, said resin,
wherein said fibers are distributed throughout said resin in a random orientation.

2. The composition of claim 1, wherein said fluoroplastic resin is polytetrafluoroethylene (PTFE).

3. The composition of claim 2, wherein said fiber is a fiber selected from the group consisting of aramids, ultrahigh molecular weight polyethylenes, polyesters, silicones, polyurethanes, and elastomers.

4. The composition of claim 2 or 3, wherein the amount by weight of said fibers is from about .0005% to about 10% of said composition.

5. The composition of claim 4, wherein said fibers have a diameter of from about 1 micron to about 50 microns.

6. The composition of claim 5, wherein said diameter is from about 5 microns to about 25 microns.

7. The composition of claim 4, wherein said fibers have a length of from about 1 mm to about 5 cm.

8. The composition of claim 7, wherein said length is from about 4 mm to about 2 cm.

9. The composition of claim 4, wherein said PTFE has a molecular weight of from about 500,000 to about 1,000,000.

10. The composition of claim 2, wherein said fibers can withstand exposure to a temperature of at least about 342° C.

11. A composite in vivo vascular graft material, conducive to perigraft tissue ingrowth and facilitating suture retention, comprising: a porous, preformed, extruded, expanded and sintered blend of biocompatible resin and biocompatible fibers, wherein said resin comprises polytetrafluorethylene (PTFE) and said fibers are chemically compatible with said PTFE and said fibers further are distributed throughout said resin in a random orientation.

12. The vascular graft material of claim 11, wherein said fibers are selected from the group consisting of aramids, polyurethanes, elastomers, polytetrafluoroethylenes expanded polytetrafluoroethylenes, and combinations thereof.

13. The vascular graft material of claim 11, wherein said aramid is an aromatic polyamide.

14. The vascular graft material of claim 11, wherein said elastomer is a perfluorinated elastomeric copolymer of tetrafluoroethylene and perfluoro (methyl vinyl ether).

15. The vascular graft material of claim 11, wherein said fibers are selected from polytetrafluorotheylenes and expanded polytetrafluoroethylenes.

16. The vascular graft material of claim 11 or 12, further characterized in that the weight to weight ratio of fibers to resin is from about 5 parts fibers per million parts resin to about 1 part fibers per 10 parts resin.

17. The vascular graft material of claim 16, wherein said fibers have a diameter of from about 1 micron to about 50 microns.

18. The vascular graft material of claim 17. wherein said diameter is from about 5 microns to about 25 microns.

19. The vascular graft material of claim 16, wherein said fibers have a length of from about 1 mm to about 5 cm.

20. The vascular graft material of claim 17, wherein said length is from about 4 mm to about 2 cm.

21. The vascular graft material of claim 16, wherein said PTFE resin has a molecular weight of from about 5,000 to about 1,000,000.

22. The vascular graft material of claim 11, wherein said fibers provide a means for retaining said sutures.

23. The vascular graft material of claim 11, wherein said material has a porosity of from about 20% to about 90%.

24. The vascular graft material of claim 23, wherein said material has a wall thickness of about 0.2 mm to about 0.8 mm.

25. The vascular graft material of claim 24, wherein said wall has a thickness of about 0.5 mm.

26. A composite in vivo vascular graft material, conducive to perigraft tissue ingrowth and facilitating suture retention, manufactured by the process comprising.

    a) mixing lubricated biocompatible fluoroplastic powders with, fibers that are chemically compatible with said PTFE,

    b) preforming said mixture,

    c) extruding said preformed mixture to form a tubular extrudate,

    d) expanding said extrudate material such that it has a porosity of about 20% to about 90%, and

    e) sintering said expanded graft material.

27. The composition of claim 26, wherein said fluoroplastic powders comprise PTFE.

28. The composition of claim 27, wherein said fiber is a fiber selected from the group consisting of aramids, ultrahigh molecular weight polyethylenes, polyesters, silicone, polyurethanes, elastomers, polytetrafluoroethylene and expanded polytetrafluoroethylene.

29. The composition of claim 28, wherein said aramid is an aromatic polyamide.

30. The composition of claim 28, wherein said elastomer is a perfluorinated elastomeric copolymer of tetrafluoroethylene and perfluoro (methyl vinyl ether).

31. The composition of claim 28, 29, or 30 wherein said fibers comprise from about 0.0005% to about 10% by weight of said mixture.

32. The composition of claim 27, wherein said lubricant comprises about 20% by weight mineral spirits.

33. The composition of claim 27, wherein said mixing step comprises tumbling of said lubricated PTFE powder while adding said fibers.

34. The composition of claim 31, wherein the extruded tube has an internal diameter corresponding to the internal diameter of said perigraft tissue to which said vascular graft will be appended.

35. The composition of claim 27, wherein said expansion is from about 110 to about 800% of said material's original length.

36. The composition of claim 35, wherein said expansion is about 400% of said material's original length.

37. The composition of claim 27, wherein said expanded material is sintered at about 350° C.

38. The composition of claim 27, wherein said material is sintered for about 3 minutes.

FIG. 1    SEM LUMEN SURFACE OF FIBERS
REINFORCED PTFE GRAFT

FIG. 2    SEM OUTSIDE SURFACE OF FIBERS
REINFORCED PTFE GRAFT

FIG. 3  SEM CROSS-SECTION OF FIBERS
REINFORCED PTFE GRAFT (A FIBER
SURROUNDED BY PTFE NODES)

SUTURES
7

PTFE NODE
4

PTFE FIBRE
3

REINFORCING
FIBER
5

LONGITUDINAL

CIRCUMFERENTIAL

RADIAL

FIG. 4

EP 0 352 972 A2